# EUROPEAN PATENT SPECIFICATION

(11) **EP 3 393 598 B1**
(45) Date of publication and mention of the grant of the patent: **11.11.2020**
(21) Application number: 16804753.8
(22) Date of filing: 29.11.2016
(51) Int. Cl.: A61Q 11/00, A61K 8/81

(54) **A COMPOSITE FOR BIOFILM CONTROL**
COMPOSIT MATERIAL ZUR BIOFILM KONTROLLE
MATERIEL COMPOSITE POUR LA CONTROLE DU BIOFILM

(30) Priority: 24.12.2015 EP 15202679
(43) Date of publication of application: 31.10.2018
(73) Proprietor: Unilever NV, 3013 AL Rotterdam (NL); Unilever PLC, London, Greater London EC4Y 0DY (GB)
(72) Inventor: DAS, Somnath, Bangalore 560 066 (IN); KUMARAN, Srikala, Bangalore 560066 (IN); MITRA, Rupak, Bangalore 560 066 (IN); PRAMANIK, Amitava, Bangalore 560 066 (IN); SARKAR, Priyanka, Bangalore 560 066 (IN)
(74) Representative: Tansley, Sally Elizabeth
(86) International application number: PCT/EP2016/079077
(87) International publication number: WO 2017/108346

(56) References cited:
- EP-A1- 0 321 650
- EP-A1- 2 241 339
- EP-A2- 0 341 662
- WO-A2-02/072020
- LEI M ET AL: "Effects of poly (sodium 4-styrene-sulfonate) on morphology of calcium carbonate particles", JOURNAL OF CRYSTAL GROWTH, ELSEVIER, AMSTERDAM, NL, vol. 294, no. 2, 4 September 2006 (2006-09-04), pages 358-366, XP028016925, ISSN: 0022-0248, DOI: 10.1016/J.JCRYSGRO.2006.06.029 [retrieved on 2006-09-04]

## Description

### Field of the invention

The invention relates to a composition, which prevents, or at least delays, the onset of biofouling of animate and inanimate surfaces.

### Background of the invention

S. *mutans* is primarily responsible for dental caries. Dental plaque is a cosmetically unacceptable condition and if left untreated, it may become a medical condition. On the other hand, caries involves demineralization of the dental enamel and of the dentin and in advanced stages it may affect the pulp space.

It is believed that dental plaque contains variety of bacteria. Plaque is a type of biofilm and biofilms are responsible for biofouling of various substrates.

The role of *S.mutans* in biofouling of the oral cavity, in particular the teeth, has been widely reported. Biofilms cause an imbalance between demineralisation and remineralisation. Biofilms contain a thin mucous layer having embedded microorganisms (for example, bacteria such as *Streptococcus mutans*). Usually biofilms contain water and some extra-cellular polymeric substances excreted by the microorganisms, such as proteins, lipids, and nucleic acids.

Biofouling is a universal phenomenon. Not only does it affect animate surfaces like teeth but it also affects inanimate surfaces like tiles, porcelain and even surfaces of things like boats and ships. Bacterial biofilms on teeth are primarily responsible for decay and oral malodour. Antimicrobial agents such as triclosan and cetyltrimethyl ammonium chloride are used in oral care compositions to prevent or at least delay the onset of biofilm formation. However, these compounds non-selectively destroy all types of bacterial flora even if they are beneficial to the body or even if they are otherwise not harmful.

Sodium polystyrene sulfonates (PSS) and some of its derivatives are known to prevent, or at least inhibit, biofilm formation especially on mammalian tooth. However, their functionality tends to diminish over a period due to high water solubility of the material.

JP2015174860A (Sunstar INC, 2015) discloses use of liposomes which contain cetylpyridinium chloride to reduce the adhesion of biofilms containing *S*. *mutans* or *S*. *aureus* to the teeth.

US20140051036 A1 (Helmholtz-Zentrum fuer Infektionsforschung GmbH) discloses tooth filling materials and dental varnishes for inhibiting formation of biofilm of of *S.mutans.* It contains an active substance which inhibits the formation of biofilms formation, a material for forming a structure for the uptake and delayed release of the active substance and a filling material system.

JP2004035416 A (NAT INST INFECTIOUS DISEASES, 2004) discloses the combined effect of chlorhexidine and hydroxyapatite against biofilms.

US2003165440 AA and US 2008/0226566 A1 (Henkel) discloses compositions which comprise low-solubility calcium salts and polyelectrolytes, and are suitable for the smoothing, restoration and remineralizing of tooth and bone tissue. The polyelectrolytes are polyacids and polybases, biopolymers or synthetic polymers. The polyelectrolytes adhere to the surface of the fine-particle calcium salts, but there is no chemical reaction. The individual molecules adsorbed on the surface are free of intermolecular linkages to one another.

WO02072020 A2 (Phycogen Inc.) discloses monomeric/polymeric compounds which exhibit anti-adhesive properties which are useful for antifouling applications. The list of polymers includes polystyrene sulphonates.

Cryst. Growth Des. 2014, 14, 6073-6083 discloses the use of *in situ* mixing of complementary polyelectrolytes as templates for controlling crystal growth of calcium carbonate. The crystals of calcium carbonate are synthesised by insitu reaction of calcium chloride with sodium carbonate. The carbonate is added through an aqueous solution of polystyrene sulphonate. This aqueous solution is contacted with another aqueous solution containing the chloride salt and a polycation. The resulting products are polyelectrolyte complexes, said to suitable for various applications, such as drug or proteins encapsulation, biosensing platform besides drug delivery and imaging.

Crystal Growth & Design, Vol. 4, No. 1, 2004 discloses fabrication of biomimetic coatings for artificial implants by deposition of organic/inorganic composite materials consisting of polyelectrolyte multilayers alternating with layers of "in situ" grown calcium phosphate crystals.

Journal of Crystal Growth 294 (2006) 358-366 discloses CaCO₃ crystals with diverse morphologies and polymorphs prepared by precipitation of calcium chloride and sodium carbonate in the presence of poly (sodium 4-styrene-sulfonate) at room temperature.

We have now determined that it is possible to inhibit, at least partially, the adhesion of bacterial biofilms on certain substrates, without adversely affecting viability of microflora present in the biofilms.

### Summary of the invention

In accordance with a first aspect is disclosed a composite material in accordance with claim 1.

All other aspects of the invention will hereinafter be explained in details.

### Detailed description of the invention

### The composite material

In accordance with a first aspect is disclosed a composite material comprising:
(i) a water-insoluble inorganic compound selected from oxide, hydroxide, phosphate or silicate of calcium, magnesium, titanium, zinc or aluminium; and,
(ii) 0.5 to 10 % by weight polystyrene sulphonate (pss) wherein at least 60 % of the total pss content is entrapped as a complex with said calcium, magnesium, titanium, zinc or aluminium within crystals of said inorganic compound.

The composite material is a compound which comprises the components mentioned under (i) and (ii) and which represent aggregates, which are microscopically heterogeneous, but macroscopically homogeneous. The insoluble inorganic compound, preferably in the form of individual crystallites or in the form of particles, comprising a majority of said crystallites, are present in associated form in the structure of the polystyrene sulphonate.

The term "insoluble" means solubility of the inorganic compound (in water) is not more than 4 X10⁻⁴ [g/100ml].

It is preferred that the insoluble inorganic compound is zinc oxide, calcium oxide, calcium carbonate, titanium oxide, aluminium silicate or magnesium carbonate. Alternatively, it is preferred that the inorganic compound is an apatite selected from hydroxyapatite, fluorapatite or chlorapatite. It is particularly preferred that the inorganic compound is hydroxyapatite.

Hydroxyapatite is the major component, and an essential ingredient of bones and teeth. It is stable at ambient temperature (25 °C) and it thermally decompose at temperatures from about 800-1200 °C.

In alkaline pH, polystyrene sulphonate forms a complex with bivalent and trivalent metal ions. In case of a bivalent metal ion, one ion can bind to two polystyrene sulphonate moieties while for a trivalent metal ion, there can be three polystyrene sulphonate moieties bound to one metal ion. In the presence of alkali, the metal precipitates as its corresponding oxide or hydroxide. If other anions such as phosphate is present in the medium, the corresponding phosphates precipitate. During such precipitation, the polystyrene sulphonate is entrapped in the matrix of the insoluble precipitate.

Sodium polystyrene sulfonates (PSS) and its derivatives are known to inhibit the formation of biofilms. However, the activity drops significantly when the material is deposited on certain surfaces, as it tends to rinse-off easily in an aqueous or water-like environment. Simple adsorption of PSS onto insoluble inorganic carriers does not increase or prolong its efficacy. Even an admixture of the two does not provide a technical effect.

It is preferred that the amount of the polystyrene sulphonate in the composite material is 0.5 to 5 % by weight of the composite.

It is particularly preferred that Zeta Potential of the composite material is in the range of -15 to -40 mV.

### Use of the composite material

In accordance with a second aspect is disclosed a composite material in accordance with the first aspect for use to inhibit adhesion of bacterial biofilm to an animate or inanimate substrate.

The animate substrate could be any suitable substrate but it preferably ate is mammalian teeth, in particular human teeth. The inanimate substrate could be any hard or soft surface, which is amenable to growth of microbiological biofilm on it. Such surfaces include ceramic, vitrified tile, glass, paper, PMMA, polystyrene or marble.

It is preferred that the bacterium is a Gram-positive bacterium.

Gram-positive bacteria give positive result in the Gram stain test by taking up or imbibing the crystal violet stain. Such bacteria appear purple under a microscope. This is because the thick peptidoglycan layer in the bacterial cell wall retains the stain even after being washed. On the other hand, Gram-negative bacteria do not retain the violet stain. Several species of bacteria can form aggregates on surfaces and such aggregates are called biofilms. The biofilm protects them against antibiotics and antibodies.

Biofilms are known to cause infections in the body. Dental plaque is an oral biofilm that adheres to the teeth and consists of multiple species of fungal and bacterial cells (such as S. *mutans*), salivary polymers and microbial extracellular products. The accumulation of microorganisms subjects the teeth and gingival tissues to high concentrations of bacterial metabolites, which if left untreated, could result in dental disease.

It is preferred that the Gram-positive bacterium belong to the genus *Streptococcus.* It is particularly preferred that the Gram-positive bacterium is *Streptococcus mutans* (*S.mutans*) which is facultative anaerobic bacterium commonly found in the oral cavity of human beings. S. *mutans* stick to the surface of teeth and subsists on a diverse group of carbohydrates. While metabolising sugar and other energy sources, the microbe produces acid that may even cause cavities.

### Use and method according to the invention

In accordance with another aspect is disclosed use of a composite material according to the first aspect to inhibit adhesion of bacterial biofilm to an animate or inanimate substrate.

The use is preferably for non-therapeutic purpose. Alternatively, the use is for therapeutic purpose. Skilled persons know differences between the two.

It is preferred that the non-therapeutic purpose is for cosmetic applications, which are meant to preserve the beauty or aesthetics of human or animal body. It is preferred that the therapeutic or the non-therapeutic purpose involves treatment of mammalian teeth, especially human teeth. Such a purpose could be achieved through any means known in the art but it is preferably achieved through cosmetic or medical/therapeutic compositions comprising an efficacious amount of the composite material in accordance with the invention, along with other conventional ingredients, which are usually present in such compositions.

In accordance with another aspect is disclosed a method of inhibiting adhesion of bacterial biofilm to an animate or inanimate substrate by contacting the substrate with a composite material of the first aspect. In one embodiment, the method is for non-therapeutic purpose. In such case, it is preferred that the non-therapeutic purpose is for cosmetic applications. Further, preferably the animate substrate is mammalian teeth. The method maintains viability of bacterial cells present in the biofilm, whilst inhibiting adhesion.

### Composition

In accordance with yet another aspect is disclosed a composition comprising a composite material of the first aspect. It is preferred that the composition comprises 1 to 50 % by weight of the composite. It is further preferred that the composition is a homecare or personal care composition. Homecare compositions include hard surface cleaners, floor cleaners, toilet cleaners, laundry products and laundry auxiliaries. Alternatively, it is preferred that the personal care composition is an oral care composition.

Preferred oral care compositions include toothpastes, which may be chalk-based or silica based (transparent). The compositions may also be in the form of mouthwash or alternatively a tablet or chewing gum or an edible film. The examples given herein are non-limiting.

The composition in general comprises a safe and effective amount of the composite along with a carrier suited for the intended application.

Preferably, the composition comprises 5 to 50 % by weight of the composite.

In accordance with yet another aspect is disclosed a composition comprising a composite material of the first aspect *for use* to inhibit adhesion of bacterial biofilm to an animate or inanimate substrate. The composition maintains, or does not adversely affect, the viability of bacterial cells present in the biofilm. It is preferred that the animate substrate is mammalian teeth. It is further preferred that the bacterium is a Gram-positive bacterium. Further, particularly the Gram-positive bacterium belong to the genus *Streptococcus.* It is particularly preferred that the Gram-positive bacterium is *Streptococcus mutans (S.mutans).*

When the composition is a mouthwash, it is preferred that the base is water. The desired efficacy can be obtained without the use of low molecular weight alcohols e.g. ethanol or isopropyl alcohol, i.e., the composition is preferably free of low molecular weight alcohols. The mouthwash composition preferably comprises 0.05 to 10 % by weight, more preferably 0.05 to 8 % by weight of a surfactant. The surfactant is preferably of the cationic, anionic, or zwitterionic class, most preferably of the cationic class. When anionic surfactant is present, it preferably is alkali or alkaline earth metal salts of alkyl sulphonic acid, fatty acid, or alkyl ether sulphate. When zwitterionic surfactant is present it is preferably chosen from betaines, sulphobetaines, hydroxyl sulphobetaines and amino carboxylates When a cationic surfactant is present it is benzalkonium chloride, alkyl pyridinium chloride or quaternary ammonium gemini surfactants.

The mouthwash composition may be used undiluted or in diluted form.

### Toothpaste

The oral care composition may be a toothpaste. When the composition is a toothpaste, the orally acceptable base includes an abrasive, which may be calcium carbonate or abrasive silica. When calcium carbonate is the abrasive, the toothpaste is in the opaque paste format. When abrasive silica is used, the toothpaste is usually a transparent gel. Toothpastes also preferably include a surfactant in 2 to 15 % by weight of the composition. Preferred surfactants are anionic or amphoteric in nature. Anionic surfactant is preferably an alkali metal sulphate, more preferably a sodium lauryl sulphate (SLS). Mixtures of anionic surfactants may also be used. The amphoteric surfactant is preferably a betaine, more preferably an alkylamidopropyl betaine (wherein the alkyl group is a linear C₁₀ about C₁₈ chain), and most preferably is cocoamidopropyl betaine (CAPB). Mixtures of amphoteric surfactants may also be used. It is preferred that the amount of surfactant is 2 to about 15 % by weight, more preferably from 2.5 to 5 % by weight of the total composition.

When calcium carbonate is the abrasive, it usually accounts for 15 to 70 % by weight, more preferably 30 to 60 % by weight of the composition.

Calcium carbonate (also known as chalk) is available in many forms and some of these forms are suitable for oral care compositions. Two commonly used forms are FGNC (fine ground natural chalk) and PCC (precipitated calcium carbonate). The composition may also include other known "non-chalk" abrasives to improve the abrasive action. Such abrasives include dicalcium phosphate dihydrate (DCPD).

It is preferred that water content of the compositions is 15 to 40 % by weight, more preferably 20 to 30 % by weight of the composition. Preferred compositions include a humectant, e.g. xylitol, glycerol or sorbitol. Glycerol and sorbitol are particularly preferred. Preferably, the compositions include 0.1 to 20 % by weight humectant.

Gel toothpastes comprise abrasive silica. They have low refractive index in the range of 1.41 to 1.47. Examples include Tixosil® 63 and 73 ex Rhone Poulenc; Sident® 10 ex Degussa; Zeodent® 113 ex Zeofinn; Zeodent® 124 ex Huber, Sorbosil® AC series supplied by Crosfield, for example Sorbosil® ACI1, Sorbosil® AC39 and Sorbosil® AC35, particularly Sorbosil® AC 77 ex Crosfield Chemicals. The amount of silica in the compositions generally ranges from 2 to 60 % by weight.

Thickening silica may also be included, usually 4 to 12 % by weight of the composition. Preferred grades are medium thickening silica such as MFIL® (ex. Madhu Silica India), TC15 (from PQ Corp UK), and Zeodent® 165 Ex. Huber, or Tixosil® 43 from Rhodia.

The compositions for any type of toothpaste (opaque or gel type) may also include an anti-caries agent, binders, thickeners, flavours, stabilizing agents, polymers, vitamins, buffers and anti-calculus agents.

Toothpowders usually have large amount of abrasives. Chalk (FGNC) is the most preferred one but PCC may also be used. Usual percentages of such abrasives are from 90 to 99.9 %, preferably 90 to 95 % by weight of the composition. Desired amount of foam is provided by including an anionic surfactant e.g. Sodium Lauryl Sulphate in the toothpowder composition. The surfactant may be incorporated from 2 to 3 % by weight of the composition.

### Product by process

In accordance with yet another aspect is disclosed a water-insoluble inorganic compound selected from oxide, hydroxide, phosphate or silicate of calcium, magnesium, titanium, zinc or aluminium; and, 0.5 to 10 % by weight polystyrene sulphonate (pss) wherein at least 60 % of the total pss content is entrapped within crystals of said inorganic compound, prepared by a process comprising the steps of:
(i) contacting an aqueous slurry of polystyrene sulphonic acid with a water-soluble acidic salt of calcium, magnesium, titanium, zinc or aluminium in an aqueous medium while maintaining pH of said medium ≤ 7;
(ii) increasing pH of said medium to 8 to 10 by addition of an alkali;
(iii) adding a precipitating anion selected from oxide, hydroxide, phosphate or silicate to said medium;
(iv) re-adjusting pH of said medium to 7 to 10 by addition of an alkali;
(v) separating the precipitate which gets formed in step (iv);
(vi) aging said precipitate at 80 to 100 °C for 2 to 8 hours; and,
(vii) drying said precipitate to get said composite.

### Second use

In accordance with yet another aspect is disclosed use of a composite material of the first aspect in the preparation of a composition to inhibit adhesion of bacterial biofilm to an animate or inanimate substrate.

### Process for preparing the composite

In accordance with yet further aspect of the invention is disclosed a process for preparing a composite material in accordance with the first aspect, comprising the steps of:
(i) contacting an aqueous slurry of polystyrene sulphonic acid with a water-soluble acidic salt of calcium, magnesium, titanium, zinc or aluminium in an aqueous medium while maintaining pH of said medium ≤ 7;
(ii) increasing pH of said medium to 8 to 10 by addition of an alkali;
(iii) adding a precipitating anion selected from oxide, hydroxide, phosphate or silicate to said medium;
(iv) re-adjusting pH of said medium to 7 to 10 by addition of an alkali;
(v) separating the precipitate which gets formed in step (iv);
(vi) aging said precipitate at 80 to 100 °C for 2 to 8 hours; and,
(vii) drying said precipitate to get said composite.

The invention will be explained in further details with the help of non-limiting examples.

### Examples

### Example 1: Preparation of a composite material according to the invention

### Materials and process:

Poly(4-styrenesulfonic acid) solution, Mw -75,000, 18 wt. % in H₂O, calcium nitrate tetrahydrate, sodium fluoride, sodium dihydrogen phosphate and sodium hydroxide all procured from Sigma Aldrich.

Approximately 14 g of polystyrene sulphonic acid (PSS) (18% w/v) solution was added to 86 g de-ionised water to get an aqueous slurry containing 2.5 g PSS. This slurry was contacted with 24 g calcium, followed by 0.025 g sodium fluoride. The pH of the medium was maintained ≤ 7. In the next step, this pH was increased to 8 by addition of 0.1 M sodium hydroxide solution. Upon addition of the alkali, the colour of the medium turned faint pink. Thereafter, 7 g (0.6 M) sodium dihydrogen phosphate was added to the medium under constant stirring where the phosphate anions served as precipitating anions. The pH of the solution dropped to ∼5 with the formation of a viscous gelatinous precipitate. In the next step, the pH was re-adjusted to 8 by addition of solution of sodium hydroxide. A white precipitate formed. The precipitate was separated from the aqueous medium by filtration. The precipitate was aged in an air oven maintained at 90 °C for 6 hours, followed by ageing at room temperature for 6 hours. Thereafter, the precipitate was air-dried and the composite material was ground finely using a standard kitchen mixer.

The composite so formed comprised water-insoluble inorganic compound which is phosphate of calcium and 0.5 to 10 % by weight polystyrene sulphonate (pss). At least 60 % of the total pss content is entrapped within crystals of the inorganic compound. The inorganic compound is an apatite (hydroxyapatite).

### Effect of particles of Example-1 on bacterial biofilms using hydroxyapatite (HA) disk as substrate

A culture of S. *mutans* [UA159] was inocculated in Tryptone Yeast Extract (TY medium) broth overnight at 37 °C in 5% carbon-di-oxide incubator. The culture was diluted in the ratio 1:20 in a low molecular weight medium (LMW i.e., TY medium filtered through 10 KDa filter), and incubated at 37 °C until they reached 0.5 OD (Optical Density, mid log phase). The mid-log phase culture was diluted to 1:250 in LMW medium containing 2 % sucrose. Two ml of the culture was added into 12 well-plate and a treated HA disk was immersed into each well and incubated overnight at 37 °C, 5 % carbon-di-oxide incubator. At the end of incubation period (20 to 22 hours) the disc was stained with erythrosine dye. The erythrosin was extracted using 0.1 N NaOH and absorbance measured at OD 527. Another set of disks were also tested for viability.

The HA disks were prepared as follows:
About 50 mg of particles of Example-1 were dispersed in 1 ml water. An HA disk was immersed in this dispersion for two minutes, followed by dip and wash thrice in sterile distilled water at the end of the contact time.

The observations are summarised in table 1.

**Table 1**

| Description of the disk | Amount of dye bound to the biofilm (mg/ml) |
|---|---|
| Control, i.e., untreated HA disk | 5.88 |
| Disk treated with composite of Example-1 | 1.23 |

The data shown in table 1 indicates that the composite of Example-1 is far more effective at inhibiting the adhesion of bacterial biofilm. This could be inferred by the amount of dye bound to the biofilm. Lesser amount of dye (bound) indicates more inhibition.

### Comparative Example-1

An experiment was done to determine whether simple coating of the polymer onto the inorganic ingredient makes it an efficacious material.

### Procedure:

An aqueous dispersion of PSS (2.5 % by weight) was prepared and its pH adjusted to 8. To this dispersion, 100 mg hydroxyapatite was added. The mix was agitated for 4 hours and aged for 6 hours at 90 °C. The particles were filtered and air-dried. They were labelled Comparative Particle-1.

The particles were subjected to thermo gravimetric analysis using a Perkin® 1 TGA analyzer. About 10 mg were heated from 25 °C to 700 °C in an inert atmosphere and the weight loss was monitored as is done in thermo gravimetry.

The observations are summarised in table 3.

**Table 2**

| Description of the particles | % weight loss |
|---|---|
| Comparative Particle-1 | 2.5 |
| Composite of Example-1 | 11.0 |

The relatively little loss in weight in the case of Comparative Particle-1 indicates that it is not a composite material. The loss also indicates that there is practically little molecular interaction between the ingredients; otherwise, the organic component would have caused more weight loss. On the other hand, the Composite of Example-1 (inside the invention) lost significant amount of the weight, which indicates that there is significantly more interaction at the molecular levels between the ingredients, i.e., the water-insoluble inorganic and the polystyrene sulphonate, which indirectly indicates at least 60 % of the total pss content is entrapped within crystals of the inorganic compound.

### Further tests conducted on the composite of Example-1

In order to find out the effect of composite of Example-1 on biofilms and bacterial cell viability, the following tests were conducted in accordance with procedure explained earlier. The observations are summarised in table 3.

**Table 3**

| Description of the test particles | Relative biofilm inhibition on HA disc (dye mg/ml) [Δ = control minus treated samples] | Bacterial cell viability on HA disk (log CFU/ml) |
|---|---|---|
| *S*. *mutans* control | 0 | 6.7 |
| Comparative Example-1 | 0.8 | 6.7 |
| Example-1 | 4.7 | 5.2 |

The observations summarised in table 3 clearly indicate a higher Δ (delta) value in the case of composite material of Example 1. This indicates more that the composite material of Example-1 inhibits adhesion of bacterial biofilm to the inanimate substrate (HA disk) to greater extent as compared to the others which were tested. On the other hand, there is greater cell viability reflected by lower log reduction value. This indicates that the composite material of Example-1 does not affect the bacteria present on the disk to the extent done by the material made in accordance with Comparative Example-1 (outside the invention).

### Efficacy of Composite of Example-1 v/s conventional antibacterial agent

This experiment compares efficacy of Composite of Example-1 with a conventional, but potent, antibacterial agent. The procedure has been described earlier. The observations are summarised in table 4.

**Table 4**

| Description of the disk | Amount of dye bound to the biofilm (mg/ml) |
|---|---|
| Control, i.e., untreated disk | 5.88 |
| Disk treated with triclosan | 6.33 |
| Disk treated with composite of Example-1 | 1.23 |

The data in table 4 clearly indicates that a composite in accordance with the invention is far more effective and much better than triclosan.

### Dose response study

In this experiment, the amount of dye bound to the biofilm (mg/ml) is measured as a function of the amount of composite of Example-1. The objective is to find out how dosage affects adhesion of bacterial biofilm to the inanimate substrate.

The procedure has been described earlier. The observations are summarised in table 5.

**Table 5**

| Description of the disk | Amount of dye bound to the biofilm (mg/ml) |
|---|---|
| Control, i.e., untreated disk | 9.48 |
| Composite of Example-1, 100 mg/ml | 1.82 |
| Composite of Example-1, 200 mg/ml | 2.45 |
| Composite of Example-1, 300 mg/ml | 1.42 |
| Composite of Example-1, 400 mg/ml | 3.20 |
| Composite of Example-1, 500 mg/ml | 3.32 |

The data clearly indicates that as compared to control, the composite material of the invention is effective at all the levels tried. However, the upward trend starting from 400 mg/ml, appears to indicate that beyond some level, which could be beyond 500 mg/ml, the effect may not be as good as the effect at comparatively lower levels.

### Homecare composition containing the composite of Example-1

10 g of composite of Example-1 were mixed with 100 g Domex® cleaner Ex. Unilever. About 0.5 ml of the resulting mix was deposited on clean glass microscopic slide (2.5 cm X12 cm). The slides were wiped clean with a tissue paper. The gloss level was measured using a gloss meter (Sheen Instruments Ltd UK, cat No. 4174) at detector angle of 20° against a black background (R=0, G=0, B=0). Reduction in gloss value is taken as indication of deposition of particles of the Composite of Example-1.

The same steps were repeated with Cif® cream, also Ex. Unilever.

The observations are summarised in table 6.

**Table 6**

| Description of the material deposited on the slide | Gloss value |
|---|---|
| Clean untreated glass | 158 |
| Cif® | 124 |
| Cif® with particles of composite of Example-1 | 106 |
| Domex® | 122 |
| Domex® with particles of composite of Example-2 | 94 |

Interpretation of the data is as follows:
The gloss value is inversely proportional to the amount of particles deposited on the slide. The difference (i.e., reduction) in gloss value as seen in the case of Cif® v/s Cif® with particles of composite of Example-1, as well as in the case of Domex® v/s Domex® with particles of composite of Example-1 clearly indicates deposition of the particles.

## Claims

1. A composite material comprising:
(i) a water-insoluble inorganic compound selected from oxide, hydroxide, phosphate or silicate of calcium, magnesium, titanium, zinc or aluminium; and,
(ii) 0.5 to 10 % by weight polystyrene sulphonate (pss) wherein at least 60 % of the total pss content is entrapped as a complex with said calcium, magnesium, titanium, zinc or aluminium within crystals of said inorganic compound.

2. A composite material as claimed in claim 1 wherein said inorganic compound is an apatite selected from is hydroxyapatite, fluorapatite or chlorapatite.

3. A composite material as claimed in claim 1 or 2 wherein the amount of said polystyrene sulphonate is 0.5 to 5 % by weight of said composite.

4. A composite material as claimed in any preceding claim 1 to 3 wherein Zeta Potential of said material is in the range of -15 to -40 mV.

5. A composite material as claimed in claim 1 for use to inhibit adhesion of bacterial biofilm to an animate or inanimate substrate.

6. A composite material *for use* as claimed in claim 5 wherein said animate substrate is mammalian teeth.

7. A composite material for use as claimed in claim 5 wherein said inanimate substrate is ceramic, vitrified tile, glass, paper, poly methyl methacrylate (PMMA), polystyrene or marble.

8. Non-therapeutic use of a composite material as claimed in claim 1 to inhibit adhesion of bacterial biofilm to an inanimate substrate.

9. A composition comprising a composite material as claimed in claim 1.

10. A composition as claimed in claim 9 comprising 1 to 50 % by weight of said composite.

11. A composition as claimed in claim 9 or 10 wherein said composition is a homecare or personal care composition.

12. A composition comprising a composite material as claimed in claim 1 *for use* to inhibit adhesion of bacterial biofilm to an animate or inanimate substrate.

13. A process for preparing a composite material as claimed in claim 1 comprising the steps of:
(i) contacting an aqueous slurry of polystyrene sulphonic acid with a water-soluble acidic salt of calcium, magnesium, titanium, zinc or aluminium in an aqueous medium while maintaining pH of said medium ≤ 7;
(ii) increasing pH of said medium to 8 to 10 by addition of an alkali;
(iii) adding a precipitating anion selected from oxide, hydroxide, phosphate or silicate to said medium;
(iv) re-adjusting pH of said medium to 7 to 10 by addition of an alkali;
(v) separating the precipitate which gets formed in step (iv);
(vi) aging said precipitate at 80 to 100 °C for 2 to 8 hours; and,
(vii) drying said precipitate to get said composite.

## Patentansprüche

1. Kompositmaterial, umfassend:
(i) eine wasserunlösliche anorganische Verbindung, ausgewählt aus Oxid, Hydroxid, Phosphat oder Silicat von Calcium, Magnesium, Titan, Zink oder Aluminium; und
(ii) 0,5 bis 10 Gewichts-% Polystyrolsulfonat (PSS), wobei mindestens 60% des gesamten PSS-Gehalts als ein Komplex mit dem genannten Calcium, Magnesium, Titan, Zink oder Aluminium innerhalb von Kristallen der anorganischen Verbindung eingeschlossen sind.

2. Kompositmaterial wie in Anspruch 1 beansprucht, wobei die anorganische Verbindung ein Apatit ist, das aus Hydroxyapatit, Fluorapatit oder Chlorapatit ausgewählt ist.

3. Kompositmaterial wie in Anspruch 1 oder 2 beansprucht, wobei die Menge des Polystyrolsulfonats 0,5 bis 5 Gewichts-% des Komposits beträgt.

4. Kompositmaterial wie in irgendeinem der vorhergehenden Ansprüche 1 bis 3 beansprucht, wobei das Zeta-Potential des Materials im Bereich von -15 bis -40 mV liegt.

5. Kompositmaterial wie Anspruch 1 beansprucht zur Verwendung zur Hemmung der Adhäsion von bakteriellem Biofilm an ein belebtes oder unbelebtes Substrat.

6. Kompositmaterial zur Verwendung wie in Anspruch 5 beansprucht, wobei das belebte Substrat Säugetierzähne ist.

7. Kompositmaterial zur Verwendung wie in Anspruch 5 beansprucht, wobei das unbelebte Substrat Keramik, verglaste Fliese, Glas, Papier, Polymethylmethacrylat (PMMA), Polystyrol oder Marmor ist.

8. Nichttherapeutische Verwendung eines Kompositmaterials wie in Anspruch 1 beansprucht zur Hemmung der Adhäsion von bakteriellem Biofilm an einem unbelebten Substrat.

9. Zusammensetzung, umfassend ein Kompositmaterial wie in Anspruch 1 beansprucht.

10. Zusammensetzung wie in Anspruch 9 beansprucht, umfassend 1 bis 50 Gewichts-% des Komposits.

11. Zusammensetzung wie in Anspruch 9 oder 10 beansprucht, wobei die Zusammensetzung eine Homecare- oder Körperpflegezusammensetzung ist.

12. Zusammensetzung, umfassend ein Kompositmaterial wie in Anspruch 1 beansprucht zur Verwendung zur Hemmung der Adhäsion von bakteriellem Biofilm an ein belebtes oder unbelebtes Substrat.

13. Verfahren zur Herstellung eines Kompositmaterials wie in Anspruch 1 beansprucht, umfassend die Schritte:
(i) Inkontaktbringen einer wässrigen Aufschlämmung von Polystyrolsulfonsäure mit einem wasserlöslichen sauren Salz von Calcium, Magnesium, Titan, Zink oder Aluminium in einem wässrigen Medium unter Beibehaltung des pH-Werts des Mediums bei ≤ 7;
(ii) Erhöhen des pH-Werts des Mediums auf 8 bis 10 durch Zugabe eines Alkalis;
(iii) Hinzufügen eines Fällungsanions, ausgewählt aus Oxid, Hydroxid, Phosphat oder Silicat, zu dem Medium;
(iv) erneutes Einstellen des pH-Werts des Mediums auf 7 bis 10 durch Zugabe eines Alkalis;
(v) Trennen des Niederschlags, der sich in Schritt (iv) gebildet hat;
(vi) Altern des Niederschlags bei 80 bis 100 °C für 2 bis 8 Stunden; und
(vii) Trocknen des Niederschlags, um das Komposit zu erhalten.

## Revendications

1. Matériau composite comprenant :
(i) un composé inorganique insoluble dans l'eau choisi parmi un oxyde, hydroxyde, phosphate ou silicate de calcium, magnésium, titane, zinc ou aluminium ; et,
(ii) 0,5 à 10 % en masse de poly(sulfonate de styrène) (pss) dans lequel au moins 60 % de la teneur totale en pss sont piégés comme un complexe avec ledit calcium, magnésium, titane, zinc ou aluminium dans des cristaux dudit composé inorganique.

2. Matériau composite selon la revendication 1, dans lequel ledit composé inorganique est une apatite choisie parmi l'hydroxyapatite, fluorapatite ou chlorapatite.

3. Matériau composite selon la revendication 1 ou 2, dans lequel la quantité dudit poly(sulfonate de styrène) est de 0,5 à 5 % en masse dudit composite.

4. Matériau composite selon l'une quelconque des revendications 1 à 3, dans lequel le potentiel Zeta dudit matériau se trouve dans l'intervalle de -15 à -40 mV.

5. Matériau composite selon la revendication 1 pour une utilisation pour inhiber l'adhérence de biofilm bactérien à un substrat animé ou inanimé.

6. Matériau composite pour une utilisation selon la revendication 5, dans lequel ledit substrat animé est une dent de mammifère.

7. Matériau composite pour une utilisation selon la revendication 5, dans lequel ledit substrat inanimé est une céramique, une tuile vitrifiée, un verre, papier, poly(méthacrylate de méthyle) (PMMA), polystyrène ou marbre.

8. Utilisation non-thérapeutique d'un matériau composite selon la revendication 1 pour inhiber l'adhérence de biofilm bactérien à un substrat inanimé.

9. Composition comprenant un matériau composite selon la revendication 1.

10. Composition selon la revendication 9 comprenant de 1 à 50 % en masse dudit composite.

11. Composition selon la revendication 9 ou 10, dans laquelle ladite composition est une composition pour le soin de la maison ou de la personne.

12. Composition comprenant un matériau composite selon la revendication 1 pour une utilisation pour inhiber l'adhérence de film biobactérien à un substrat animé ou inanimé.

13. Procédé de préparation d'un matériau composite selon la revendication 1 comprenant les étapes de :
(i) mise en contact d'une suspension aqueuse de poly(acide styrènesulfonique) avec un sel acide soluble dans l'eau de calcium, magnésium, titane, zinc ou aluminium dans un milieu aqueux tout en maintenant le pH dudit milieu ≤ 7 ;
(ii) augmentation du pH dudit milieu à de 8 à 10 par addition d'un alcali ;
(iii) addition d'un anion de précipitation choisi parmi un oxyde, hydroxyde, phosphate ou silicate audit milieu ;
(iv) ré-ajustement du pH dudit milieu à de 7 à 10 par addition d'un alcali ;
(v) séparation du précipité qui est formé dans l'étape (iv) ;
(vi) vieillissement dudit précipité à de 80 à 100°C pendant de 2 à 8 heures ; et,
(vii) séchage dudit précipité pour obtenir ledit composite.
